## Europäisches Patentamt

## European Patent Office

## Office européen des brevets

(19)

(11) Veröffentlichungsnummer: **0 202 474**
**B1**

(12) **EUROPÄISCHE PATENTSCHRIFT**

(45) Veröffentlichungstag der Patentschrift: **16.01.91**

(51) Int. Cl.⁵: $A\ 61\ F\ 5/04$, $A\ 61\ F\ 13/10$

(21) Anmeldenummer: **86105216.5**

(22) Anmeldetag: **16.04.86**

(54) Schultermanschette.

(30) Priorität: **19.04.85 DE 8511756 u**

(43) Veröffentlichungstag der Anmeldung:
**26.11.86 Patentblatt 86/48**

(45) Bekanntmachung des Hinweises auf die
Patenterteilung:
**16.01.91 Patentblatt 91/03**

(84) Benannte Vertragsstaaten:
**AT BE CH DE FR GB IT LI NL SE**

(56) Entgegenhaltungen:
**FR-A- 840 015**
**FR-A-2 504 387**
**US-A-3 906 944**
**US-A-4 347 848**

(73) Patentinhaber: **Habermeyer, Peter, Dr.**
**Oberföhringer Strasse 27**
**D-8000 München 81 (DE)**

(72) Erfinder: **Habermeyer, Peter, Dr.**
**Oberföhringer Strasse 27**
**D-8000 München 81 (DE)**

(74) Vertreter: **Dipl.-Phys.Dr. Manitz Dipl.-Ing., Dipl.-
W.-Ing. Finsterwald Dipl.-Ing. Grämkow Dipl.-
Chem.Dr. Heyn Dipl.-Phys. Rotermund
Morgan, B.Sc.(Phys.) Robert-Koch-Strasse 1
D-8000 München 22 (DE)**

EP 0 202 474 B1

**Beschreibung**

Die Erfindung betrifft eine Schultermanschette, insbesondere zur Verwendung nach Verletzungen und Luxationen der Schulter und bei schmerzhaften Erkrankungen des Schultergelenks.

Ein Verband zur Behandlung von Verletzungen der Schulter ist aus der FR-A-2 504 387 bekannt. Dieser Verband ist nach Art eines Hemdes ausgebildet und umschließt unter Aussparung des unverletzten Schulterbereichs den Oberkörper sowie den durch die Verletzung beeinträchtigten Arm des jeweiligen Patienten. Der durch die Verletzung beeinträchtigte Arm kann auch in einem hemdinnenseitig oder hemdaußenseitig fixierbaren Ärmel aufgenommen sein, wobei diese Fixierung des Ärmels über Klettverschlüsse und taschenförmige, mit dem Hemd verbundene Aufnahmen erfolgt. Auf der der verletzten Schulter gegenüberliegenden Seite des Oberkörpers ist der hemdenartige Verband geteilt und mittels mehrerer Verschlußelemente verschließbar.

Ein Verband dieser Art führt unvermeidbar zu unerwünschten Schwitzeffekten im gesamten Oberkörperbereich, verursacht sowohl beim Anlegen als auch beim Wechseln des Verbandes aufgrund der oft weitgehenden Bewegungsunfähigkeit des verletzten Armes erhebliche Schmerzen und kann überdies aufgrund des erforderlichen straffen Umschließens des gesamten Oberkörpers Beklemmungsgefühle verursachen.

Aufgabe der Erfindung ist es, eine Schultermanschette so auszubilden, daß sie problemfrei angelegt und gewechselt werden kann, dem Patienten die jeweils zulässige Bewegungsfreiheit läßt und zusätzlich stabilisierende und auch die Einleitung von Andrückkräften in den Bereich der verletzten Schulter gewährleistende Eigenschaften besitzt.

Gelöst wird die gestellte Aufgabe im wesentlichen dadurch, daß die Schultermanschette besteht aus einem schlauchförmigen, radial dehnbaren, zur ärmelartigen Umspannung des Oberarmbereichs und zur Überspannung des Schulterbereichs, nicht aber der Anordnung am Oberkörper unterhalb des Armes der entsprechenden Schulter bestimmten Manschettenteil und aus zwei schulterseitig damit verbundenen, brust- bzw. rückenüberspannenden und an ihren freien Enden miteinander verbindbaren Haltebändern.

Um zusätzlich zu den stabilisierenden Eigenschaften speziell zur Schmerzlinderung und zur Förderung des Heilungsprozesses beizutragen, ist nach einer vorteilhaften Ausgestaltung der Erfindung zumindest der Manschettenteil mit wenigstens einer Tasche zur Aufnahme und Fixierung eines flachen Kühl- oder Wärmebeutels versehen.

In vielen Fällen führt insbesondere die gezielte Anwendung von Kälte bei Schulterverletzungen und Luxationen und bei anderen Erkrankungen des Schultergelenks zu einer wesentlichen Schmerzlinderung, wobei die erfindungsgemäße Ausgestaltung der Manschette die Anbringung eines Kältebeutels nicht nur erleichtert, sondern vor allem auch sicherstellt, daß aufgrund der vorgegebenen Positionierung der Tasche die Kälteeinwirkung auch im jeweils optimalen Bereich erfolgt, und zwar auch dann, wenn diese Kältebehandlung vom Patienten selbst durchgeführt wird.

Die jeweilige Tasche ist vorzugsweise durch ein auf den Manschettenteil aufgesetztes, aus einem elastischen Material bestehendes Gewebestück gebildet, so daß durch die Eigenelastizität dieses Materials stets eine gute Fixierung des jeweils eingebrachten Beutels sichergestellt ist.

Nach einer Ausführungsvariante besteht der Manschettenteil aus einem doppelwandigen Schlauch, wobei zumindest ein Teilbereich dieses doppelwandigen Schlauches als Tasche ausgebildet und zum Beispiel durch dreiseitig vorgesehene Steppnähte begrenzt ist.

Gemäß einer weiteren Ausführungsform kann das die Tasche bildende Gewebestück mit dem Manschettenteil zumindest zum Teil über Klettverschlüsse verbunden sein, wodurch das Einbringen bzw. Wechseln der jeweiligen Kältetaschen erleichtert und der Ort der Anbringung insbesondere dann, wenn die Gesamtbefestigung der Tasche über Klettverschlüsse erfolgt, variabel vorgegeben werden kann.

Zur Erleichterung des Anlegens und des Wechselns der Manschette sind an den freien Enden der Haltebänder auch zur Spannungseinstellung und Fixierung dienende Klettverschlußelemente vorgesehen, wobei eine doppelseitige Ausbildung dieser Verschlüsse das Anlegen der Manschette an der linken und an der rechten Schulter gestattet.

Die Haltebänder können einteilig mit dem Manschettenteil ausgebildet oder an den Manschettenteil angesetzt sein. Von wesentlichem Vorteil ist es, diese Haltebänder mit einer ihre Flachform stabilisierenden Versteifung, z.B. in Form eines Schaumstoffstreifens, zu versehen.

Weitere vorteilhafte Ausführungen der Erfindung sind in Unteransprüchen angegeben.

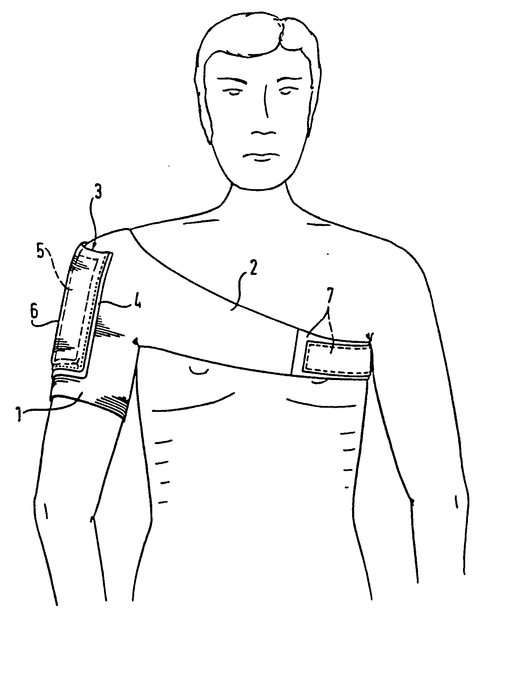

Ein Ausführungsbeispiel wird nachfolgend unter Bezugnahme auf die Zeichnung erläutert.

Die einzige Figur der Zeichnung zeigt eine schematische Darstellung einer einem Patienten angelegten Schultermanschette in Vorderansicht.

Nach der Zeichnung ist eine aus einem strumpfartigen Manschettenteil 1 und Haltebändern 2 bestehende Schultermanschette einem Patienten in der Weise angelegt, daß sie sich ausgehend vom Oberarm über die Schulter erstreckt und in ihrer Soll-Lage mittels der Brust bzw. Rücken überspannenden Haltebänder 2 fixiert ist.

Die Haltebänder sind dabei so ausgebildet, daß sie ihre Flachform im gespannten Zustand behalten, wodurch der Tragekomfort erhöht und auch das Anlegen der Manschette erleichtert wird.

Bevorzugt sind die Haltebänder einteilig mit dem Manschettenteil 1 ausgebildet und mit einer ihre Flachform sicherstellenden Versteifung versehen. An den freien Enden der Haltebänder 2 sind bevorzugt Klettverschlußelemente 7 vorgesehen, welche einerseits eine flächige und damit nicht drückende Verbindung und andererseits eine bequeme Einstellung der gewünschten Spannung ermöglichen.

Am Manschettenteil 1 ist zumindest eine Tasche 3 ausgebildet, die zweckmäßigerweise aus einem auf den Manschettenteil 1 aufgesetzten Gewebestück 6 besteht, das dreiseitig mit dem Manschettenteil 1 verbunden ist.

Diese Tasche 3 dient zur Aufnahme eines Kältebeutels oder ggf. auch eines Wärmebeutels, und sie stellt aufgrund ihrer vorgegebenen Anordnung sicher, daß die Einwirkung von Kälte bzw. Wärme auf den Patienten gezielt an der richtigen Stelle erfolgt.

Das die Tasche 3 bildende Gewebestück besitzt vorzugsweise eine Radialelastizität, die der des Manschettenteils 1 entspricht. Dabei ist es möglich, die Fixierung des Gewebestücks 6 am Manschettenteil 1 über lösbare Haltestreifen 4, insbesondere in Form von Klettverschlußstreifen vorzunehmen, um auf diese Weise das Einbringen des jeweiligen Beutels und das Wechseln weiter zu erleichtern.

Lösbare Haltestreifen können an zwei oder drei Seiten der Tasche 3 vorgesehen sein.

Durch die Erfindung wird es möglich, auf einfache und auch vom Patienten durchführbare Weise neben der elastischen Stützfunktion auf das Gelenk eine gezielte Anwendung von insbesondere Kälte vorzunehmen und auf diese Art vor allem schmerzlindernde Wirkungen zu erzielen, die wesentlich auch dazu beitragen, den Einsatz von entsprechenden Medikamenten zu verringern.

## Patentansprüche

1. Schultermanschette, insbesondere zur Verwendung nach Verletzungen und Luxationen der Schulter und bei schmerzhaften Erkrankungen des Schultergelenks, dadurch gekennzeichnet, daß die Schultermanschette besteht aus einem schlauchförmigen, radial dehnbaren, zur ärmelartigen Umspannung des Oberarmbereichs und zur Überspannung des Schulterbereichses, nicht aber zur Anordnung am Oberkörper unterhalb des Armes der entsprechenden Schalter bestimmten Manschettenteil (1) und aus zwei schulterseitig damit verbundenen, brust- bzw. rückenüberspannenden und an ihren freien Enden miteinander verbindbaren Haltebändern (2).

2. Schultermanschette nach Anspruch 1, dadurch gekennzeichnet, daß zumindest der Manschettenteil (1) mit wenigstens einer Tasche (3) zur Aufnahme und Fixierung eines flachen Kühl- oder Wärmebeutels (5) versehen ist.

3. Schultermanschette nach Anspruch 2, dadurch gekennzeichnet, daß die Tasche (3) durch ein auf den Manschettenteil (1) aufgesetztes, insbesondere aus einem elastischen Material bestehendes Gewebestück (6) gebildet ist.

4. Schultermanschette nach Anspruch 2, dadurch gekennzeichnet, daß der Manschettenteil (1) aus einem doppelwandigen Schlauch besteht und zumindest ein Teilbereich dieses doppelwandigen Schlauches als Tasche (3) ausgebildet ist.

5. Schultermanschette nach Anspruch 3, dadurch gekennzeichnet, daß das die Tasche (3) bildende Gewebestück (6) mit dem Manschettenteil (1) zumindest zum Teil über Klettverschlüsse verbunden ist.

6. Schultermanschette nach Anspruch 1, dadurch gekennzeichnet, daß an den freien Enden der Haltebänder (2) zur Spannungseinstellung und Fixierung Klettverschlußelemente (7) vorgesehen sind.

7. Schultermanschette nach einem der vorhergehenden Ansprüche, dadurch gekennzeichnet, daß die Haltebänder (2) einteilig mit dem Manschettenteil (1) ausgebildet sind.

8. Schultermanschette nach einem der vorhergehenden Ansprüche, dadurch gekennzeichnet, daß die Haltebänder (2) mit einer ihre Flachform stabilisierenden Versteifung versehen sind.

9. Schultermanschette nach Anspruch 8, dadurch gekennzeichnet, daß die Versteifung aus einem Schaumstoff besteht.

10. Schultermanschette nach einem oder mehreren der Ansprüche 7 bis 9, dadurch gekennzeichnet, daß die Haltebänder (2) aus einem in Längsrichtung im wesentlichen nicht dehnbaren, schlauchförmigen Material bestehen, in dem die Versteifung als Einlage angeordnet ist.

## Revendications

1. Bandage pour épaule, notamment destiné à être utilisé après des blessures ou luxations de l'épaule, ainsi que dans le cas de lésions douloureuses de l'articulation de l'épaule, caractérisé en ce que le bandage pour épaule se compose d'une partie formant manchon (1) de forme tubulaire, extensible radialement et destinée à enserrer à la manière d'une manche la région du bras et à recouvrir la région de l'épaule. mais non pas à être mise en place sur le buste au-dessous du bras de l'épaule considérée, ainsi que de deux bandes de maintien (2) reliées, au niveau de l'épaule, à la partie formant manchon, recouvrant respectivement la poitrine et le dos et pouvant être reliées l'une à l'autre au niveau de leurs extrémités libres.

2. Bandage pour épaule selon la revendication 1, caractérisé en ce qu'au moins la partie formant manchon (1) est munie d'au moins une poche (3) destinée à recevoir et à maintenir une vessie plate (5) froide ou chaude.

3. Bandage pour épaule selon la revendication 2, caractérisé en ce que la poche (3) est constituée par une pièce de tissu (6) notamment en un matériau élastique, rapportée sur la partie formant manchon (1).

4. Bandage pour épaule selon la revendication 2, caractérisé en ce que la partie formant manchon (1) se compose d'un tube à double paroi et en ce qu'au moins une partie de ce tube à double paroi est réalisée sous forme de poche (3).

5. Bandage pour épaule selon la revendication 3, caractérisé en ce que la pièce de tissu (6) formant la poche (3) est reliée à la partie formant manchon (1), au moins en partie, par l'intermédiaire de bandes agrippantes.

6. Bandage pour épaule selon la revendication 1, caractérisé en ce que des éléments (7) de bandes agrippantes sont prévus aux extrémités libres des bandes de maintien (2) et sont destinés au réglage de la tension et à la fixation.

7. Bandage pour épaule selon l'une des revendications précédentes, caractérisé en ce que les bandes de maintien (2) sont réalisées d'un seul tenant avec la partie formant manchon (1).

8. Bandage pour épaule selon l'une des revendications précédentes, caractérisé en ce que les bandes de maintien (2) sont munies d'un renfort stabilisant leur forme plate.

9. Bandage pour épaule selon la revendication 8, caractérisé en ce que le renfort est constitué par une mousse.

10. Bandage pour épaule selon l'une ou plusieurs des revendications 7 à 9, caractérisé en ce que les bandes de maintien (2) sont réalisées en un matériau tubulaire ne pouvant pratiquement pas s'allonger dans la direction longitudinale et dans lequel le renfort est disposé sous forme d'insert.

**Claims**

1. Shoulder bandage, in particular for use with injuries and dislocations of the shoulder and painful conditions of the shoulder joint, characterised in that the shoulder bandage comprises a hose-like radially extensible cuff part (1) for sleeve-like tensioned engagement around the upper arm region and over the shoulder region, but not however for arrangement on the upper body beneath the arm of the corresponding shoulder, and of two holding bands (2) which are connected therewith at the shoulder side and which engage under tension around the breast and back respectively and are connectable together at their free ends.

2. Shoulder bandage in accordance with claim 1, characterised in that at least the cuff part (1) is provided with at least one pocket (3) for receiving and fixing a flat cooling bag or heating bag (5).

3. Shoulder bandage in accordance with claim 2, characterised in that the pocket (3) is formed by a piece of fabric, in particular a piece of fabric formed of elastic material, which is mounted onto the cuff part (1).

4. Shoulder bandage in accordance with claim 2, characterised in that the cuff part (1) comprises a double-walled hose and is formed as a pocket in at least one region of this double-walled hose.

5. Shoulder bandage in accordance with claim 3, characterised in that the piece of fabric (6) forming the pocket (3) is connected with the cuff part (1) at least in part via burr closures (interlocking hook and loop connectors such as Velcro).

6. Shoulder bandage in accordance with claim 1, characterised in that burr closure elements (7) are provided at the free ends of the holding bands (2) for adjustment of the tension and fixation.

7. Shoulder bandage in accordance with one of the preceding claims, characterised in that the holding bands (2) are formed in one piece with the cuff part (1).

8. Shoulder bandage in accordance with one of the preceding claims, characterised in that the holding bands (2) are provided with stiffening which stabilise their flat shape.

9. Shoulder bandage in accordance with claim 8, characterised in that the stiffening comprises a foam material.

10. Shoulder bandage in accordance with one or more of the claims 7 to 9, characterised in that the holding bands (2) comprise a hose-like material which is substantially non-extensible in the longitudinal direction in which the stiffening is arranged as an insert.